(19) Europäisches Patentamt
European Patent Office
Office européen des brevets

(11) Veröffentlichungsnummer: **0 363 883 A1**

(12) **EUROPÄISCHE PATENTANMELDUNG**

(21) Anmeldenummer: **89118755.1**

(22) Anmeldetag: **09.10.89**

(51) Int. Cl.5: **C07D 405/12 , A61K 31/44 , //(C07D405/12,311:00,213:00), (C07D405/12,311:00,237:00), (C07D405/12,311:00,239:00), (C07D405/12,311:00,241:00)**

(30) Priorität: **14.10.88 DE 3835011**

(43) Veröffentlichungstag der Anmeldung: **18.04.90 Patentblatt 90/16**

(84) Benannte Vertragsstaaten: **AT BE CH DE ES FR GB GR IT LI LU NL SE**

(71) Anmelder: **MERCK PATENT GESELLSCHAFT MIT BESCHRÄNKTER HAFTUNG**
**Frankfurter Strasse 250 Postfach 4119**
**D-6100 Darmstadt(DE)**

(72) Erfinder: **Gericke, Rolf, Dr.**
**Mozartstrasse 19**
**D-6104 Seeheim(DE)**
Erfinder: **Baumgarth, Manfred, Dr.**
**Sachsenstrasse 53**
**D-6100 Darmstadt(DE)**
Erfinder: **Lues, Ingeborg, Dr.**
**Paul-Wagner Strasse 13**
**D-6100 Darmstadt(DE)**
Erfinder: **De Peyer, Jacques, Dr.**
**Zinsgutstrasse 14**
**D-6102 Pfungstadt(DE)**
Erfinder: **Bergmann, Rolf, Dr.**
**Birkenhag 36**
**D-6101 Reichelsheim(DE)**

(54) **Chromanderivate.**

(57) Die Erfindung betrifft neue Chromanderivate der Formel I

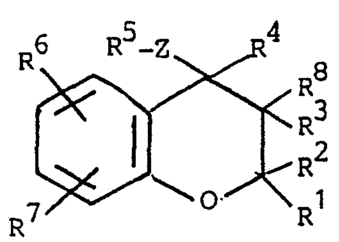

I

worin $R^1$, $R^2$, $R^3$, $R^4$ $R^5$ $R^6$ $R^7$ $R^8$ und Z die in Patentanspruch 1 angegebene Bedeutungen haben, sowie ihre Salze zeigen Wirkungen auf das cardiovaskuläre System und können verwendet werden zur Behandlung bzw. Prophylaxe von Herzinsuffizienz, Angina pectoris, Bluthochdruck, Inkontinenz und Alopezie.

EP 0 363 883 A1

## Chromanderivate

Die Erfindung betrifft neue Chromanderivate der Formel I

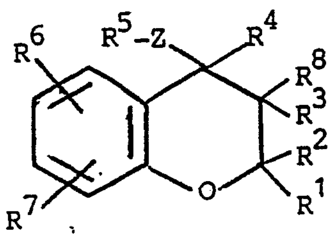

I

worin

$R^1$ A,

$R^2$ und $R^8$ jeweils H oder A,

$R^1$ und $R^2$ zusammen auch Alkylen mit 3-6 C-Atomen,

$R^3$ H, OH, OA oder OAc,

$R^4$ H,

$R^3$ und $R^4$ zusammen auch eine Bindung,

$R^5$ einen unsubstituierten oder ein- oder zweifach durch A, F, Cl, Br, J, OH, OA, OAc, SH, $NO_2$, $NH_2$, AcNH, HOOC und/oder AOOC substituierten Pyridyl-, Pyridazinyl-, Pyrimidinyl-,Pyrazinyl-, Oxo-dihydro-pyridyl-, Oxo-dihydro-pyridazinyl-, Oxo-dihydro-pyrimidinyl- oder Oxo-dihydro-pyrazinyl-rest,

$R^6$ und $R^7$ jeweils H, A, HO, AO, CHO, ACO, ACS, HOOC, AOOC, AO-CS, ACOO, A-CS-0, Hydroxyalkyl mit 1-6 C-Atomen, Mercaptoalkyl mit 1-6 C-Atomen, $NO_2$, $NH_2$, NHA, $NA_2$, CN, F, Cl, Br, J, $CF_3$, ASO, $ASO_2$, AO-SO, AO-$SO_2$, AcNH, AO-CO-NH, $H_2NSO$, HANSO, $A_2NSO$, $H_2NSO_2$, $HANSO_2$, $A_2NSO_2$, $H_2NCO$, HANCO, $A_2NCO$, $H_2NCS$, HANCS, $A_2NCS$, ASONH, $ASO_2NH$, AOSONH, $AOSO_2NH$, ACO-alkyl, Nitroalkyl, Cyanalkyl, A-C(=NOH) oder A-C(=$NNH_2$),

Z O, S, oder NH,

A Alkyl mit 1-6 C-Atomen,

alkyl Alkylen mit 1-6 C-Atomen und

Ac Alkanoyl mit 1-8 C-Atomen oder Aroyl mit 7-11 C-Atomen

bedeuten;

sowie deren Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen. So zeigen sie Wirkungen auf das cardiovaskuläre System, wobei in der Regel bei niedrigeren Dosen ein selektiver Angriff am Coronarsystem, bei höheren ein blutdrucksenkender Effekt beobachtet werden kann. Am Coronarsystem treten z.B. Widerstandsabnahme und Flußzunahme auf, wobei der Einfluß auf die Herzfrequenz gering bleibt. Weiterhin zeigen die Verbindungen eine relaxierende Wirkung auf verschiedene glattmuskuläre Organe (Gastrointestinaltrakt, Respirationssystem und Uterus). Die Wirkungen der Verbindungen können mit Hilfe an sich bekannter Methoden ermittelt werden, wie sie z.B. in der EP-A1-76075, der EP-A1-173848 oder der AU-A-45547/85 (Derwent Farmdoc Nr. 86081769) sowie von K.S. Meesmann et al., Arzneimittelforschung 25 (11), 1975, 1770-1776, angegeben sind. Als Versuchstiere eignen sich z.B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

In den angegebenen Formeln bedeutet A eine vorzugsweise unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

Falls $R^1$ und $R^2$ zusammen Alkylen bedeuten, so ist die Alkylengruppe vorzugsweise unverzweigt, im einzelnen bevorzugt $-(CH_2)_n-$, wobei n 3, 4, 5 oder 6 bedeutet.

Die Gruppe "alkyl" steht vorzugsweise für $-CH_2-$ oder $-CH_2CH_2-$.

Ac ist vorzugsweise Alkanoyl mit 1-6, insbesondere 1 ,2, 3 oder 4 C-Atomen, im einzelnen bevorzugt Formyl oder Acetyl, weiterhin bevorzugt Propionyl, Butyryl, Isobutyryl, Pentanoyl oder Hexanoyl, ferner bevorzugt Benzoyl, o-, m- oder p-Toluyl, 1- oder 2-Naphthoyl.

$R^1$ und $R^2$ sind vorzugsweise jeweils Alkyl, insbesondere jeweils Methyl oder Ethyl, bevorzugt jeweils Methyl, weiterhin sind $R^1$ und $R^2$ zusammen bevorzugt $-(CH_2)_4$-oder $-(CG_2)_5-$.

Falls $R^4$ H bedeutet, ist $R^3$ bevorzugte OH, ferner bevorzugt O-CHO oder O-$COCH_3$.

$R^5$ ist bevorzugte 6-Hydroxy-3-pyridazinyl (= 1,6-Dihydro-6-oxo-3-pyridazinyl) oder 2-Hydroxy-4-pyridyl (= 1,2-Dihydro-2-oxo-4-pyridyl), ferner bevorzugt unsubstituierten 2-, 3-oder 4-Pyridyl, 2-, 4- oder 5-Pyrimidinyl, 3-, 4- oder 5-Pyridazinyl oder Pyrazinyl,

Hydroxy-pyridyl wie 3-, 4-, 5- oder 6-Hydroxy-2-pyridyl, 2-, 4- oder 5-Hydroxy-3-pyridyl, 3-Hydroxy-4-pyridyl, 2-Hydroxy-5-pyridyl; Hydroxy-pyridazinyl wie 4- oder 5-Hydroxy-3-pyridazinyl, 3-, 5- oder 6-Hydroxy-4-pyridazinyl; Hydroxy-pyrimidinyl wie 4- oder 5-Hydroxy-2-pyrimidinyl, 2-, 5- oder 6-Hydroxy-4-pyrimidinyl, 2-oder 4-Hydroxy-5-pyrimidinyl; Hydroxy-pyrazinyl wie 3-, 5- oder 6-Hydroxy2-pyrazinyl; Dihydro-alkyl-oxo-pyridyl wie 1,2-Dihydro-1-methyl-2-oxo-3-, -4-, -5- oder -6-pyridyl, 1,2-Dihydro-1ethyl-2-oxo-3-, -4-, -5- oder -6-pyridyl; Dihydro-alkyloxo-pyridazinyl wie 1,6-Dihydro-1-methyl-6-oxo-3-, -4-oder -5-pyridazinyl, 1,6-Dihydro-1-ethyl-6-oxo-3-, -4-oder -5-pyridazinyl; Alkoxy-pyridyl wie 3-, 4-, 5- oder 6-Methoxy-2-pyridyl, 2-, 4- oder 5-Methoxy-3-pyridyl, 2-oder 3-Methoxy-4-pyridyl, 2-Methoxy-5-pyridyl, 2- oder 3-Ethoxy-4-pyridyl; Alkoxy-pyridazinyl wie 4-, 5- oder 6-Methoxy-3-pyridazinyl, 4-, 5- oder 6-Ethoxypyridazinyl, 3-, 5- oder 6-Methoxy-4-pyridazinyl, 3-, 5- oder 6-Ethoxy-4-pyridazinyl; Alkoxy-pyrimidinyl wie 4- oder 5-Methoxy-2-pyrimidinyl, 2-, 5- oder 6-Methoxy-4-pyrimidinyl, 2- oder 4-Methoxy-5-pyrimidinyl; Alkoxy-pyrazinyl wie 3-, 5- oder 6-Methoxy-2-pyrazinyl; Amino-pyridyl wie 3-, 4-, 5- oder 6-Aminopyridyl, 2-, 4- oder 5-Amino-3-pyridyl, 2- oder 3-Amino-4-pyridyl, 2-Amino-5-pyridyl; Amino-pyridazinyl wie 4-, 5- oder 6-Amino-3-pyridazinyl, 3-, 5- oder 6-Amino-4-pyridazinyl; Amino-pyrimidinyl wie 4- oder 5-Amino-2-pyrimidinyl, 2-, 5- oder 6-Amino-4-pyrimidinyl, 2- oder 4-Amino-5-pyrimidinyl; Amino-pyrazinyl wie 3-, 5- oder 6-Amino-2-pyrazinyl; Mercapto-pyridyl wie 3-, 4-, 5- oder 6-Mercapto-2-pyridyl, 2-, 4- oder 5-Mercapto-3-pyridyl, 2- (= 1,2-Dihydro-2-thioxo-4-pyridyl) oder 3-Mercapto-4-pyridyl, 2-Mercapto-5-pyridyl; Mercapto-pyridazinyl wie 4-, 5- oder 6-Mercapto-3-pyridazinyl (= 1,6-Dihydro-6-thioxo-3-pyridazinyl), 3-, 5- oder 6-Mercapto-4-pyridazinyl; Mercaptopyrimidinyl wie 4- oder 5-Mercapto-2-pyrimidinyl, 2-, 5-oder 6-Mercapto-4-pyrimidinyl, 2- oder 4-Mercapto-5-pyrimidinyl; Mercapto-pyrazinyl wie 3-, 5- oder 6-Mercapto-2-pyrazinyl.

Diejenigen Reste $R^5$, die eine einem Ring-N-Atom benachbarte Hydroxy- oder Mercaptogruppe enthalten, können auch in der tautomeren Lactam- oder Thio-lactam-form vorliegen, wie oben in Einzelfällen angegeben.

In $R^6$ und $R^7$ bedeuten vorzugsweise:

A: Methyl, ferner Ethyl;
AO: Methoxy, ferner Ethoxy;
ACO: Acetyl, ferner Propionyl;
ACS: Thioacetyl, ferner Thiopropionyl;
AOOC: Methoxycarbonyl, ferner Ethoxycarbonyl;
AO-CS: Methoxy-thiocarbonyl, ferner Ethoxythiocarbonyl;
ACOO: Acetoxy, ferner Propionoxy;
ACSO: Thio(no)acetoxy, ferner Thio(no)propionoxy;
Hydroxyalkyl: Hydroxymethyl oder 1- oder 2-Hydroxyethyl;
Mercaptoalkyl: Mercaptomethyl oder 1- oder 2-Mercaptoethyl;
NHA: Methylamino, ferner Ethylamino;
$NA_2$: Dimethylamino, ferner Diethylamino;
ASO: Methylsulfinyl, ferner Ethylsulfinyl;
$ASO_2$: Methylsulfinyl, ferner Ethylsulfonyl;
AO-SO: Methoxy-sulfinyl, ferner Ethoxy-sulfinyl;
AO-$SO_2$: Methoxy-sulfonyl, ferner Ethoxy-sulfonyl;
Ac-NH: Acetamido, ferner Formamido, Propionamido oder Benzamido;
AO-CO-NH: Methoxycarbonylamino, ferner Ethoxycarbonylamino;
HANSO: Methylaminosulfinyl, ferner Ethylaminosulfinyl
$A_2NSO$: Dimethylaminosulfinyl, ferner Diethylaminosulfinyl;
$HANSO_2$: Methylaminosulfonyl, ferner Ethylaminosulfonyl;
$A_2NSO_2$: Dimethylaminosulfonyl, ferner Diethylaminosulfonyl;
HANCO: N-Methylcarbamoyl, ferner N-Ethylcarbamoyl;
$A_2NOC$: N,N-Dimethylcarbamoyl, ferner N,N-Diethylcarbamoyl;

HANCS: N-Methyl-thiocarbamoyl, ferner N-Ethylthiocarbamoyl;
$A_2NCS$: N,N-Dimethyl-thiocarbamoyl, ferner N,N-Diethyl-thiocarbamoyl;
ASONH: Methylsulfinylamino, ferner Ethylsulfinylamino;
$ASO_2NH$: Methylsulfonylamino, ferner Ethylsulfonylamino;
AOSONH: Methoxysulfinylamino, ferner Ethoxysulfinylamino;
$AOSO_2NH$: Methoxysulfonylamino, ferner Ethoxysulfonylamino;
ACO-alkyl: 2-Oxopropyl, 2-Oxobutyl, 3-Oxobutyl, 3-Oxopentyl;
Nitroalkyl: Nitromethyl, 1- oder 2-Nitroethyl;
Cyanalkyl: Cyanmethyl, 1- oder 2-Cyanethyl;
A-C(= NOH): 1-Oximinoethyl, ferner 1-Oximinopropyl;
A-C(= $NNH_2$): 1-Hydrazonoethyl, ferner 1-Hydrazonopropyl.

Die Reste $R^6$ und $R^7$ stehen vorzugsweise in 6- und 7-Stellung des Chromansystems. Sie können jedoch auch in 5-und 6-, 5- und 7-, 5- und 8-, 6- und 8- sowie 7- und 8-Stellung stehen.

Von den Resten $R^6$ und $R^7$ ist vorzugsweise der eine H, während der andere von H verschieden ist. Dieser andere Rest steht vorzugsweise in 6-Stellung, aber auch in 5-, 7- oder 8-Stellung, und ist vorzugsweise CN oder $NO_2$, ferner bevorzugte CHO, ACO (insbesondere Acetyl), AOOC (insbesondere Methoxycarbonyl oder Ethoxycarbonyl), ACOO (insbesondere Acetoxy), weiterhin bevorzugt F, Cl, Br, J, $CF_3$, $H_2NCO$, $H_2NCS$ oder $NH_2$.

Der Rest $R^8$ ist vorzugsweise H, weiterhin bevorzugt Methyl oder Ethyl.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nach stehenden Formeln Ia bis Ii ausgedrückt werden, die der Formel I entsprechen und worin die nicht näher bezeichneten Reste die bei der Formel I angegebene Bedeutung haben, worin jedoch

in Ia $R^1$ und $R^2$ jeweils A bedeuten;
in Ib $R^1$ und $R^2$ jeweils $CH_3$ bedeuten;
in Ic $R^1$ und $R^2$ zusammen Alkylen mit 3-6 C-Atomen bedeuten;
in Id $R^5$ einen unsubstituierten oder einen durch eine OH-Gruppe substituierten Pyridyl-, Pyridazinyl-, Pyrimidinyl-oder Pyrazinyl-rest oder einen durch A substituierten Oxo-dihydro-pyridyl-oder Oxo-dihydro-pyridazinyl-rest bedeutet;
in Ie $R^5$ 2-Hydroxy-4-pyridyl oder 6-Hydroxy-3-pyridazinyl bedeutet;
in If $R^5$ 6-Hydroxy-3-pyridazinyl bedeutet;
in Ig $R^1$ und $R^2$ jeweils $CH_3$ oder zusammen $-(CH_2)_4-$oder $-(CH_2)_5-$; $R^5$ einen unsubstituierten oder einen durch eine OH-Gruppe substituierten Pyridyl-, Pyridazinyl-, Pyrimidinyl-oder Pyrazinyl-rest oder einen durch A substituierten Oxo-dihydro-pyridyl-oder Oxo-dihydro-pyridazinyl-rest;
$R^8$ H oder $CH_3$ und
Z O, S oder NH bedeutet;
in Ih $R^1$ und $R^2$ jeweils $CH_3$;
$R^5$ 2-Hydroxy-4-pyridyl, 6-Hydroxy-3-pyridazinyl oder 1,6-Dihydro-1-methyl6-oxo-3-pyridazinyl und
Z O bedeuten;
in Ii $R^1$ und $R^2$ jeweils $CH_3$;
$R^5$ 6-Hydroxy-3-pyridazinyl und
Z O bedeuten.

Weiterhin sind bevorzugt Verbindungen der Formeln I′ sowie Ia′ bis Ii′, die den Formeln I sowie Ia bis Ii entsprechen, worin jedoch jeweils zusätzlich $R^3$, H, OH, OCHO oder $OCOCH_3$ und $R^4$ H bedeuten, insbesondere solche Verbindungen der Formeln I′ sowie Ia′ bis Ii′, worin jeweils zusätzlich $R^3$ OH und $R^4$ H bedeuten.

Weiterhin sind bevorzugt Verbindungen der Formeln I″ sowie Ia″ bis Ii″, die den Formeln I sowie Ia bis Ii entsprechen, worin jedoch jeweils zusätzlich $R^3$ und $R^4$ zusammen eine Bindung bedeuten.

Ferner sind bevorzugt Verbindungen der Formeln I, I′, I″, Ia bis Ii, Ia′ bis Ii′ sowie Ia″ bis Ii″, worin jeweils zusätzlich

(a) $R^6$ von H verschieden ist und
$R^7$ H bedeutet;
(b) $R^6$ von H verschieden ist und in 6-Stellung steht und
$R^7$ H bedeutet;
(c) $R^6$ $NO_2$, CN, CHO, ACO, HOOC, AOOC, ACOO, F, Cl, Br, J, $CF_3$, $H_2NCO$, $H_2NCS$ oder $NH_2$ und
$R^7$ H bedeutet;

(d) $R^6$ $NO_2$, CN, CHO, ACO, HOOC, AOOC, ACOO, F, Cl, Br, J, $CF_3$, $H_2NCO$, $H_2NCS$ oder $NH_2$ bedeutet und in 6-Stellung steht und
$R^7$ H bedeutet;
(e) $R^6$ $NO_2$, CN, CHO, $CH_3CO$, $CH_3OOC$,$C_2H_5OOC$ oder $CH_3COO$ und
$R^7$ H bedeutet;
(f) $R^6$ $NO_2$, CN, CHO, $CH_3CO$, $CH_3OOC$,$C_2H_5OOC$ oder $CH_3COO$ bedeutet und in 6-Stellung steht und
$R^7$ H bedeutet;
(g) $R^6$ $NO_2$ oder CN und
$R^7$ H bedeutet;
(h) $R^6$ $NO_2$ oder CN bedeutet und in 6-Stellung steht und
$R^7$ H bedeutet;
(i) $R^6$ CN und
$R^7$ H bedeutet;

(j) $R^6$ CN bedeutet und in 6-Stellung steht und
$R^7$ H bedeutet.

Insbesondere sind bevorzugt Verbindungen der Formeln I, I′, I″, Ia bis Ii, Ia′ bis Ii′, Ia″ bis Ii″ sowie der übrigen vorstehend als bevorzugt gekennzeichneten Gruppen von Verbindungen, worin zusätzlich $R^8$ $CH_3$ bedeutet.

Im übrigen haben vor- und nachstehend die Reste $R^1$ bis $R^8$, A, "alkyl" und Ac die bei Formel I angegebenen Bedeutungen, wenn nicht ausdrücklich etwas anderes angegeben ist.

Gegenstand der Erfindung ist ferner ein Verfahren zur Herstellung von Chromanderivaten der Formel I, dadurch gekennzeichnet, daß man ein Chroman der Formel II

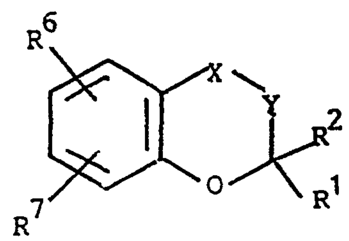

II

worin
X-Y

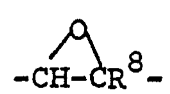

oder -CHE-$CR^3R^8$- und
E Cl, Br, J oder eine reaktionsfähig veresterte OH-Gruppe bedeuten und
$R^1$, $R^2$, $R^3$, $R^6$, $R^7$ und $R^8$ die bei Formel I angegebenen Bedeutungen haben,
mit einer Verbindung der Formel III
$R^5$-ZH III
worin $R^5$ und Z die bei Formel I angegebenen Bedeutungen haben,
oder mit einem ihrer reaktionsfähigen Derivate umsetzt
und/oder daß man eine Verbindung der Formel I, worin $R^3$ OH und $R^4$ H bedeutet, dehydratisiert und/oder daß man in einer Verbindung der Formel I einen oder mehrere der Reste $R^3$, $R^5$, $R^6$ und/oder $R^7$ in andere Reste $R^3$, $R^5$, $R^6$ und/oder $R^7$ umwandelt und/oder daß man eine basische Verbindung der Formel I durch Behandeln mit einer Säure in eines ihrer Säureadditionssalze umwandelt.

Die Verbindungen der Formel I werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier

nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden die Verbindungen der Formel I durch Reaktion von Verbindungen der Formel II mit Verbindungen der Formel III hergestellt, zweckmäßig in Gegenwart eines inerten Lösungsmittels bei Temperaturen zwischen etwa 0 und 150 °.

Ausgangsstoffe der Formel II mit

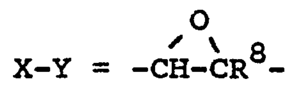

(3,4-Epoxy-chromane) sind bevorzugt.

Die Ausgangsstoffe II und III sind in der Regel bekannt (vgl. z.B. DE-OS 37 26 261). Sofern sie nicht bekannt sind, können sie nach an sich bekannten Methoden hergestellt werden. So sind die Ausgangsstoffe der Formel II

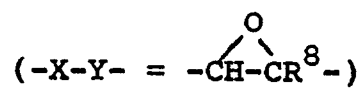

erhältlich durch Umsetzung von 2-Hydroxyacetophenonen der Formel 2-HO-$R^6R^7C_6$-$H_2$-$COCH_3$ mit Ketonen der Formel $R^1$-CO-$R^2$ zu entsprechenden 4-Chromanonen der Formel IVa

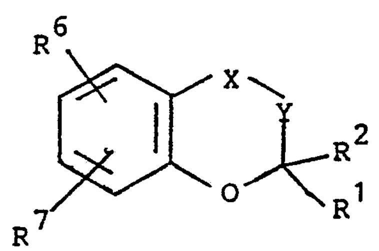

IVa -X-Y- = $-CO-CH_2-$
IVb -X-Y- = $-CO-C(=CH-R^9)-$
IVc -X-Y- = $-CHOH-CHR^8-$
IVd -X-Y- = $-CH=CR^8-$
IVe -X-Y- = $-CHBr-CR^8OH-$

gegebenenfalls Kondensation mit Aldehyden der Formel $R^9$-CHO ($R^9$ = Alkyl mit 1-5 C-Atomen) zu 3-Alkyliden-4-chromanonen der Formel IVb, Reduktion z.B. mit $NaBH_4$ zu Chromanolen der Formel IVc, Dehydratisierung z.B. mit p-Toluolsulfonsäure, zu Chromenen der Formel IVd und Oxydation, z.B. mit 3-Chlorperbenzoesäure. Die letztgenannte Oxydation kann auch mehrstufig erfolgen. So kann man, z.B. mit N-Bromsuccinimid in wäßriger Lösung zunächst die Bromhydrine der Formel IVe herstellen und aus diesen anschließend mit einer Base, z.B. Natronlauge, HBr abspalten.

Man kann die Chromene der Formel IVd auch erhalten durch Kondensation von Salicylaldehyden der Formel 2-HO-$R^6R^7$-$C_6H_2$-CHO mit Ketonen der Formel $R^1$-CO-$CH_2$-$R^8$ zu Hydroxyketonen der Formel 2-HO-$R^6R^7C_6H_2$-CH=$CR^8$-CO-$R^1$, Umsetzung mit Organo-Li-Verbindungen der Formel $R^2$-Li und nachfolgende Hydrolyse zu Diolen der Formel 2-HO-$R^6R^7C_6H_2$-CH=$CR^8$-$CR^1R^2$-OH und Cyclisierung unter Wasserabspaltung.

In Verbindungen der Formel II (-X-Y- = -CHE-$CR^3R^8$-) kommen als "reaktionsfähig veresterte OH-Gruppen" insbesondere die Ester mit Alkylsulfonsäuren (worin die Alkylgruppe 1-6 C-Atome enthält) oder mit Arylsulfonsäuren (worin die Arylgruppe 6-10 C-Atome enthält) in Betracht. Diese Verbindungen sind erhältlich aus den 4-Chromanolen der Formel IVc durch Umsetzung mit einem anorganischen Säurehalogenid wie $PCl_3$, $PBr_3$, $SOCl_2$ oder $SOBr_2$ oder mit einem Sulfonsäurechlorid wie Methan- oder p-Toluolsulfonsäurechlorid.

Als reaktionsfähige Derivate von III eignen sich die entsprechenden Salze, z.B. die Na- oder K-Salze, die auch in situ entstehen können.

Es ist zweckmäßig, in Gegenwart einer Base zu arbeiten. Als Basen eignen sich z.B. Alkalimetall- oder Erdalkalimetall-hydroxide, -carbonate, -alkoholate, -hydride oder auch -amide wie NaOH, KOH, $Ca(OH)_2$, $Na_2CO_3$, $K_2CO_3$, Na-oder K-methylat, -ethylat oder tert.-butylat, NaH, KH, $CaH_2$, $NaNH_2$, $KNH_2$, ferner

organische Basen wie Triethylamin oder Pyridin, die auch im Überschuß angewendet werden können und dann gleichzeitig als Lösungsmittel dienen.

Als inerte Lösungmittel eignen sich insbesondere Alkohole wie Methanol, Ethanol, Isopropanol, n-Butanol oder tert.-Butanol; Ether wie Diethylether, Diisopropylether, Tetra hydrofuran oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Dimethylformamid (DMF), Dimethylacetamid oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Das Epoxid II

(X-Y = 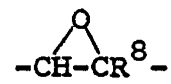 )

kann auch in situ hergestellt werden, z.B. durch Einwirkung einer Base auf das entsprechende Bromhydrin IVe.

Eine besonders bevorzugte Arbeitsweise besteht darin, daß man einen Alkohol (z.B. Ethanol) als Lösungsmittel verwendet und eine organische Base (z.B. Pyridin) hinzusetzt, wobei man zweckmäßig etwa 0,5 bis 20 Std. kocht.

Eine Verbindung der Formel I, worin $R^3$ = OH und $R^4$ = H ist, kann durch Behandeln mit einem Dehydratisierungsmittel in eine Verbindung der Formel I, worin $R^3$ und $R^4$ zusammen eine Bindung bedeuten, umgewandelt werden. Das gelingt z.B. durch Einwirkung einer der angegebenen Basen, z.B. NaH, in einem der angegebenen Lösungsmittel, z.B. DMSO, bei Temperaturen zwischen 0 und 150°. Insbesondere Verbindungen, worin Z = S ist, können auf diese Weise dehydratisiert werden.

Weiterhin kann man in einer Verbindung der Formel I einen oder mehrere der Reste $R^3$, $R^5$, $R^6$ und/oder $R^7$ in andere Reste $R^3$, $R^5$, $R^6$ und/oder $R^7$ umwandeln.

Beispielsweise ist es möglich, daß man ein H-Atom mittels einer Halogenierung durch ein Halogenatom oder mittels einer Nitrierung durch eine Nitrogruppe ersetzt und/oder eine Nitrogruppe zu einer Aminogruppe reduziert und/oder eine Amino- oder Hydroxygruppe alkyliert oder acyliert und/oder eine Cyangruppe (z.B. mit HCl in Wasser/Methanol bei 20-100°) in eine Carboxylgruppe oder (z.B. mit Raney-Nickel in Wasser/Essigsäure/Pyridin in Gegenwart von Natriumphosphat) in eine Formylgruppe oder (z.B. mit KOH in tert.-Butanol) in eine Carbamoylgruppe oder (z.B. mit $H_2S$ in Pyridin/Triethylamin) in eine Thiocarbamoylgruppe umwandelt und/oder eine -CO-NH-Gruppe (z.B. mit $P_2S_5$ oder mit Lawesson-Reagenz in Toluol) in eine -CS-NH- bzw. -C(SH)=N-Gruppe umwandelt.

Eine Nitrierung gelingt unter üblichen Bedingungen, z.B. mit einem Gemisch aus konzentrierter $HNO_3$ und konzentrierter $H_2SO_4$ bei Temperaturen zwischen 0 und 30°. Falls mindestens einer der Substituenten $R^6$ und $R^7$ eine elektronegative Gruppe wie CN oder $NO_2$ bedeutet, erfolgt die Nitrierung überwiegend am Rest $R^5$; andernfalls erhält man in der Regel Gemische, bei denen die Nitrogruppen am Rest $R^5$ oder am Chromanring stehen können.

Analoges gilt für die Halogenierung, die z.B. mit elementarem Chlor oder Brom in einem der üblichen inerten Lösungsmittel bei Temperaturen zwischen etwa 0 und 30° durchgeführt werden kann.

Eine primäre oder sekundäre Aminogruppe und/oder eine OH-Gruppe kann durch Behandeln mit alkylierenden Mitteln in die entsprechende sekundäre oder tertiäre Aminogruppe und/oder Alkoxygruppe umgewandelt werden. Als alkylierende Mittel eignen sich z.B. Verbindungen der Formeln A-Cl, A-Br oder A-J oder entsprechende Schwefelsäure-oder Sulfonsäureester wie Methylchlorid, -bromid, -jodid, Dimethylsulfat, Methyl-p-toluolsulfonat. Ferner kann man z.B. eine oder zwei Methylgruppen mit Formaldehyd in Gegenwart von Ameisensäure einführen. Die Alkylierung wird zweckmäßig in Gegenwart oder Abwesenheit eines der genannten inerten Lösungemittel, z.B. DMF, bei Temperaturen zwischen etwa 0° und etwa 120° vorgenommen, wobei auch ein Katalysator zugegen sein kann, vorzugsweise eine Base wie Kalium-tert.-butylat oder NaH.

Als acylierende Mittel zur Acylierung von Amino- oder Hydroxygruppen eignen sich zweckmäßig die Halogenide (z.B. Chloride oder Bromide) oder Anhydride von Carbonsäuren der Formel Ac-OH, z.B. Acetanhydrid, Propionylchlorid, Isobutyrylbromid, Ameisensäure/Essigsäureanhydrid, Benzoylchlorid. Der Zusatz einer Base wie Pyridin oder Triethylamin bei der Acylierung ist möglich. Man acyliert zweckmäßig in

Gegenwart oder Abwesenheit eines inerten Lösungsmittels, z.B. eines Kohlenwasserstoffs wie Toluol, eines Nitril wie Acetonitril, eines Amids wie DMF oder eines Überschusses einer tertiären Base wie Pyridin oder Triethylamin bei Temperaturen zwischen etwa 0° und etwa 160°, vorzugsweise zwischen 20° und 120°. Eine Formylierung gelingt auch mit Ameisensäure in Gegenwart von Pyridin.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasser stoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsaure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z.B. Pikrate, können zur Aufreinigung der Verbindungen der Formel I verwendet werden.

Die Verbindungen der Formel I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Racemate, beispielsweise durch Umkristallisieren aus inerten Lösungsmitteln, in reiner Form isolieren kann. So haben z.B. Verbindungen der Formel I, worin $R^1$ = $R^2$, $R^3$ = OH und $R^4$ = H ist, zwei chirale Zentren; bei der Herstellung durch Reaktion von II mit III entsteht jedoch ganz überwiegend nur ein Racemat mit trans-Stellung der Substituenten $R^3$ = OH und $R^5$-Z. Erhaltene Racemate können, falls erwünscht, nach an sich bekannten Methoden mechanisch, chemisch oder biochemisch in ihre Enantiomeren getrennt werden. So können aus dem Racemat durch Umsetzung mit einem optisch aktiven Trennmittel Diastereomere gebildet werden. Als Trennmittel für basische Verbindungen der Formel I eignen sich z.B. optisch aktive Säuren, wie die D- und L-Formen von Weinsäure, Dibenzoylweinsäure, Diacetylweinsäure, Camphansaure, Camphersulfonsäuren, Mandelsäure, Äpfelsäure oder Milchsäure. Carbinole (I, $R^3$ = OH) können ferner mit Hilfe chiraler Acylierungsreagenzien, z.B. den angegebenen Säuren, insbesondere (+)- oder (-)-Camphansäure oder (+)-oder (-)-Campher-10-sulfonsäure, oder mit D- oder L-α-Methylbenzylisocyanat, verestert und dann getrennt werden (vgl. EP-A1-120428). Die verschiedenen Formen der Diastereomeren können in an sich bekannter Weise, z.B. durch fraktionierte Kristallisation, getrennt, und die Enantiomeren der Formel I können in an sich bekannter Weise aus den Diastereomeren in Freiheit gesetzt werden. Enantiomerentrennungen gelingen ferner durch Chromatographie an optisch-aktiven Trägermaterialien.

Die Verbindungen der Formel I und ihre physiologisch unbedenklichen Salze können zur Herstellung pharmazeutischer Zubereitungen verwendet werden, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human-oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z. B. zur Herstellung von Injektionspräparaten verwendet werden. Insbesondere für die topische Anwendung kommen auch liposomale Zubereitungen in Betracht. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks-

und/oder Aromastoffe enthalten. Sie können, falls erwünscht, auch einen oder mehrere weitere Wirkstoffe enthalten, z. B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems, insbesondere dekompensierter Herzinsuffizienz, Angina pectoris, Arrhythmie, peripheren oder cerebralen Gefäßerkrankungen, sowie Krankheitszuständen, die mit Bluthochdruck verbunden sind, ferner von Erkrankungen, die mit Veränderungen der nicht-vaskulären Muskulatur verbunden sind, z.B. Asthma, Inkontinenz der Harnblase.

Dabei werden die erfindungsgemäßen Substanzen in der Regel in Analogie zu bekannten Antianginosa bzw. Blutdrucksenkern, z. B. Nicorandil oder Cromakalim verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die Verbindungen der Formel I und ihre Salze eignen sich, insbesondere bei topischer Anwendung, ferner zur Behandlung der Alopecia areata. Hierfür werden insbesondere pharmazeutische Zubereitungen verwendet, die zur topischen Behandlung der Kopfhaut geeignet und die oben genannt sind. Sie enthalten etwa 0,005 bis 10, bevorzugt 0,5 bis 3 Gew.% mindestens einer Verbindung der Formel I und/oder mindestens eines ihrer Salze. Im übrigen können diese Verbindungen gegen Alopezie in Analogie zu den Angaben in der WO 88/00822 verwendet werden.

In den nachfolgenden Beispielen bedeutet "übliche Aufarbeitung":

Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

Vor- und nachstehend sind alle Temperaturen in ° C angegeben. $[\alpha] = [\alpha]_D^{20}$ in Methanol.

Beispiel 1

Ein Gemisch von 20,1 g 2,2-Dimethyl-3,4-epoxy-6-cyanchroman ("IIa"), 14 g 2-hydroxypyridin (1H-2-Pyridon), 7 ml Pyridin und 70 ml Ethanol wird 2 Std. gekocht. Man kühlt ab, filtriert ab, konzentriert das Filtrat und chromatographiert den Rückstand an Kieselgel. Mit Diethylether/Ethylacetat (1:1) erhält man 2,2-Dimethyl-4-(2-pyridyl-oxy)-6-cyan-3-chromanol, F. 102-103°.

Analog (Kochzeiten bis 15 Std.) erhält man:

2,2,3-Trimethyl-4-(2-pyridyl-oxy)-6-cyan-3-chromanol, F. 105-107°
2,2-Tetramethylen-4-(2-pyridyl-oxy)-6-cyan-3-chromanol, F. 126-127°
2,2-Pentamethylen-4-(2-pyridyl-oxy)-6-cyan-3-chromanol, F. 108-110°
2,2-Dimethyl-4-(2-pyridyl-oxy)-6-nitro-3-chromanol
2,2-Dimethyl-4-(2-pyridyl-oxy)-6-brom-3-chromanol
2,2-Dimethyl-4-(2-pyridyl-oxy)-6-methoxycarbonyl-3-chromanol
2,2-Dimethyl-4-(3-pyridyl-oxy)-6-cyan-3-chromanol, F. 202-204°
2,2,3-Trimethyl-4-(3-pyridyl-oxy)-6-cyan-3-chromanol
2,2-Dimethyl-4-(4-pyridyl-oxy)-6-cyan-3-chromanol, F. 193-196°
2,2,3-Trimethyl-4-(4-pyridyl-oxy)-6-cyan-3-chromanol
2,2-Dimethyi-4-(3-pyridazinyl-oxy)-6-cyan-3-chromanol
2,2,3-Trimethyl-4-(3-pyridazinyl-oxy)-6-cyan-3-chromanol
2,2-Dimethyl-4-(4-pyrimidinyl-oxy)-6-cyan-3-chromanol, F. 93-105°
2,2,3-Trimethyl-4-(4-pyrimidinyl-oxy)-6-cyan-3-chromanol
2,2-Dimethyl-4-(2-pyrazinyl-oxy)-6-cyan-3-chromanol, F 103-105°
2,2,3-Trimethyl-4-(2-pyrazinyl-oxy)-6-cyan-3-chromanol.

Beispiel 2

Ein Gemisch von 20,1 g IIa, 11,1 g 2,4-Dihydroxypyridin, 8 ml Pyridin und 400 ml Ethanol wird 15 Std. gekocht. Man dampft ein, extrahiert mit Ethylacetat, wäscht die organische Phase mit verdünnter Salzsäure, dann mit Wasser, trocknet, dampft ein und erhält 2,2-Dimethyl4-(2-Hydroxy-4-pyridyl-oxy)-6-cyan-3-chromanol ("A"), F. 249-249,5° (aus Ethanol).

Analog erhält man
mit 2,4-Dihydroxypyridin:
2,2,3-Trimethyl-4-(2-hydroxy-4-pyridyl-oxy)-6-cyan-3-chromanol, F. 198-200°
2,2-Tetramethylen-4-(2-hydroxy-4-pyridyl-oxy)-6-cyan-3-chromanol
2,2-Pentamethylen-4-(2-hydroxy-4-pyridyl-oxy)-6-cyan-3-chromanol
2,2-Dimethyl-4-(2-hydroxy-4-pyridyl-oxy)-6-nitro-3-chromanol, F. 224-226°
2,2,3-Trimethyl-4-(2-hydroxy-4-pyridyl-oxy)-6-nitro-3-chromanol
2,2-Dimethyl-4-(2-hydroxy-4-pyridyl-oxy)-6-brom-3-chromanol
2,2,3-Trimethyl-4-(2-hydroxy-4-pyridyl-oxy)-6-brom-3-chromanol
2,2-Dimethyl-4-(2-hydroxy-4-pyridyl-oxy)-6-methoxycarbonyl-3-chromanol, F. 251-252°
2,2,3-Trimethyl-4-(2-hydroxy-4-pyridyl-oxy)-6-methoxycarbonyl-3-chromanol;
mit 2,3-Dihydroxypyridin:
2,2-Dimethyl-4-(2-hydroxy-3-pyridyl-oxy)-6-cyan-3-chromanol, F. 262-265°;
2,2,3-Trimethyl-4-(2-hydroxy-3-pyridyl-oxy)-6-cyan-3-chromanol;
mit 2,5-Dihydroxypyridin:
2,2-Dimethyl-4-(2-hydroxy-5-pyridyl-oxy)-6-cyan-3-chromanol, F. 256-258°;
2,2,3-Trimethyl-4-(2-hydroxy-5-pyridyl-oxy)-6-cyan-3-chromanol;
mit 4,6 Dihydroxypyrimidin:
2,2-Dimethyl-4-(6-hydroxy-4-pyrimidinyl-oxy)-6-cyan-3-chromanol, F. 235-237°
2,2,3-Trimethyl-4-(6-hydroxy-4-pyrimidinyl-oxy)-6-cyan-3-chromanol; mit 3,6-Dihydroxypyridazin:
2,2-Dimethyl-4-(6-hydroxy-3-pyridazinyl-oxy)-6-cyan-3-chromanol ("B"), F. 255-256°
2,2,3-Trimethyl-4-(6-hydroxy-3-pyridazinyl-oxy)-6-cyan-3-chromanol, F. 236-239°
2,2-Tetramethylen-4-(6-hydroxy-3-pyridazinyl-oxy)-6-cyan-3-chromanol
2,2-Pentamethylen-4-(6-hydroxy-3-pyridazinyl-oxy)-6-cyan-3-chromanol, kein F. bis 275°
2,2-Dimethyl-4-(6-hydroxy-3-pyridazinyl-oxy)-6-nitro-3-chromanol, kein F. bis 260°
2,2,3-Trimethyl-4-(6-Hydroxy-3-pyridazinyl-oxy)-6-nitro-3-chromanol, F. 223-225°
2,2-Dimethyl-4-(6-hydroxy-3-pyridazinyl-oxy)-6-brom-3-chromanol, F. 257-259°
2,2,3-Trimethyl-4-(6-hydroxy-3-pyridazinyl-oxy)-6-brom-3-chromanol
2,2-Dimethyl-4-(6-hydroxy-3-pyridazinyl-oxy)-6-methoxycarbonyl-3-chromanol, F. 242°
2,2,3-Trimethyl-4-(6-hydroxy-3-pyridazinyl-oxy)-6-methoxycarbonyl-3-chromanol.

Beispiel 3

Ein Gemisch von 20,1 g IIa, 11,1 g 2-Mercaptopyridin, 6,6 ml Pyridin und 265 ml Ethanol Wird 3 Std. gekocht. Man konzentriert und kristallisiert das erhaltene 2,2-Dimethyl-4-(2-pyridyl-thio)-6-cyan-3-chromanol aus Diisopropylether, F. 101-103°.

Analog erhält man:
2,2,3-Trimethyl-4-(2-pyridyl-thio)-6-cyan-3-chromanol
2,2-Dimethyl-4-(3-pyridyl-thio)-6-cyan-3-chromanol
2,2,3-Trimethyl-4-(3-pyridyl-thio)-6-cyan-3-chromanol
2,2-Dimethyl-4-(4-pyridyl-thio)-6-cyan-3-chromanol
2,2,3-Trimethyl-4-(4-pyridyl-thio)-6-cyan-3-chromanol
2,2-Dimethyl-4-(2-hydroxy-4-pyridyl-thio)-6-cyan-3-chromanol
2,2,3-Trimethyl-4-(2-hydroxy-4-pyridyl-thio)-6-cyan-3-chromanol
2,2-Dimethyl-4-(6-hydroxy-3-pyridazinyl-thio)-6-cyan-3-chromanol
2,2,3-Trimethyl-4-(6-hydroxy-3-pyridazinyl-thio)-6-cyan-3-chromanol
2,2-Dimethyl-4-(6-mercapto-3-pyridazinyl-thio)-6-cyan-3-chromanol
2,2,3-Trimethyl-4-(6-mercapto-3-pyridazinyl-thio)-6-cyan-3-chromanol.

Beispiel 4

Ein Gemisch von 2 g IIa, 1,11 g 2-Mercaptopyridin, 60 ml DMSO und 0,3 g NaH (80%ig) wird 6 Std. bei

20° gerührt und wie üblich aufgearbeitet. Man erhält 2,2-Dimethyl-4-(2-pyridyl-thio)-6-cyan-3-chromen, F. 110-112°.

Analog erhält man:

2,2,3-Trimethyl-4-(2-pyridyl-thio)-6-cyan-3-chromen
2,2-Dimethyl-4-(3-pyridyl-thio)-6-cyan-3-chromen
2,2,3-Trimethyl-4-(3-pyridyl-thio)-6-cyan-3-chromen
2,2-Dimethyl-4-(4-pyridyl-thio)-6-cyan-3-chromen
2,2,3-Trimethyl-4-(4-pyridyl-thio)-6-cyan-3-chromen
2,2-Dimethyl-4-(2-hydroxy-4-pyridyl-thio)-6-cyan-3-chromen
2,2,3-Trimethyl-4-(2-hydroxy-4-pyridyl-thio)-6-cyan-3-chromen
2,2-Dimethyl-4-(6-hydroxy-3-pyridazinyl-thio)-6-cyan-3-chromen
2,2,3-Trimethyl-4-(6-hydroxy-3-pyridazinyl-thio)-6-cyan-3-chromen
2,2-Dimethyl-4-(6-mercapto-3-pyridazinyl-thio)-6-cyan-3-chromen
2,2,3-Trimethyl-4-(6-mercapto-3-pyridazinyl-thio)-6-cyan-3-chromen.

Beispiel 5

Analog Beispiel 1 erhält man aus IIa und 2-Amino-3-hydroxypyridin das 2,2-Dimethyl-4-(3-hydroxy-2-pyridylamino)-6-cyan-3-chromanol, F. 207-208,5°.

Analog erhält man:

2,2,3-Trimethyl-4-(3-hydroxy-2-pyridyl-amino)-6-cyan-3-chromanol
2,2-Dimethyl-4-(2-hydroxy-4-pyrimidinyl-amino)-6-cyan-3-chromanol, kein F. bis 280°
2,2,3-Trimethyl-4-(2-hydroxy-4-pyrimidinyl-amino)-6-cyan-3-chromanol
2,2-Dimethyl-4-(2-hydroxy-4-pyridyl-amino)-6-cyan-3-chromanol
2,2,3-Trimethyl-4-(2-hydroxy-4-pyridyl-amino)-6-cyan-3-chromanol
2,2-Dimethyl-4-(2-hydroxy-4-pyridyl-amino)-6-nitro-3-chromanol
2,2,3-Trimethyl-4-(2-hydroxy-4-pyridyl-amino)-6-nitro-3-chromanol
2,2-Dimethyl-4-(2-hydroxy-4-pyridyl-amino)-6-brom-3-chromanol
2,2,3-Trimethyl-4-(2-hydroxy-4-pyridyl-amino)-6-brom-3-chromanol
2,2-Dimethyl-4-(6-hydroxy-3-pyridazinyl-amino)-6-cyan-3-chromanol
2,2,3-Trimethyl-4-(6-hydroxy-3-pyridazinyl-amino)-6-cyan-3-chromanol
2,2-Dimethyl-4-(6-hydroxy-3-pyridazinyl-amino)-6-nitro-3-chromanol
2,2,3-Trimethyl-4-(6-hydroxy-3-pyridazinyl-amino)-6-nitro-3-chromanol
2,2-Dimethyl-4-(6-hydroxy-3-pyridazinyl-amino)-6-brom-3-chromanol
2,2,3-Trimethyl-4-(6-hydroxy-3-pyridazinyl-amino)-6-brom-3-chromanol.

Analog erhält man aus 1,6-Dihydro-3-amino-1-methyl-6-pyridazinon:

2,2-Dimethyl-4-(1,6-dihydro-6-oxo-3-pyridazinyl-amino)-6-cyan-3-chromanol
2,2,3-Trimethyl-4-(1,6dihydro-6-oxo-3-pyridazinyl-amino)-6-cyan-3-chromanol
2,2-Dimethyl-4-(1,6-dihydro-6-oxo-3-pyridazinyl-amino)-6-nitro-3-chromanol
2,2,3-Trimethyl-4-(1,6-dihydro-6-oxo-3-pyridazinyl-amino)-6-nitro-3-chromanol
2,2-Dimethyl-4-(1,6-dihydro-6-oxo-3-pyridazinyl-amino)-6-brom-3-chromanol
2,2,3-Trimethyl-4-(1,6-dihydro-6-oxo-3-pyridazinyl-amino)-6-brom-3-chromanol.

Beispiel 6

Eine Lösung von 2,66 g 2,2-Dimethyl-4-brom-6-cyan-chroman (F. 89-92°; erhältlich durch Reduktion von 2,2-Dimethyl-6-cyan-4-chromanon mit $NaBH_4$ in $CH_3OH$ zu öligem 2,2-Dimethyl-6-cyan-4-chromanol und Umsetzung mit $PBr_3$ in Toluol bei 20°) und 2,5 g Pyridazin-3,6-diol in 70 ml DMSO wird mit 1,2 g 80%igem NaH versetzt und 3 Tage bei 20° gerührt. Nach üblicher Aufarbeitung erhält man 2,2-Dimethyl-4-(6-hydroxy-3-pyridazinyl-oxy)-6-cyan-chroman, F. 221-224°.

Analog erhält man:

2,2,3-Trimethyl-4-(6-hydroxy-3-pyridazinyl-oxy)-6-cyanchroman
2,2-Dimethyl-4-(6-hydroxy-3-pyridazinyl-oxy)-6-brom-chroman
2,2,3-Trimethyl-4-(6-hydroxy-3-pyridazinyl-oxy)-6-brom-chroman
2,2-Dimethyl-4-(6-hydroxy-3-pyridazinyl-oxy)-6-nitro-chroman
2,2,3-Trimethyl-4-(6-hydroxy-3-pyridazinyl-oxy)-6-nitro-chroman

2,2-Dimethyl-4-(2-hydroxy-4-pyridyl-oxy)-6-cyan-chroman
2,2,3-Trimethyl-4-(2-hydroxy-4-pyridyl-oxy)-6-cyan-chroman
2,2-Dimethyl-4-(2-hydroxy-4-pyridyl-oxy)-6-brom-chroman
2,2,3-Trimethyl-4-(2-hydroxy-4-pyridyl-oxy)-6-brom-chroman
2,2-Dimethyl-4-(2-hydroxy-4-pyridyl-oxy)-6-nitro-chroman
2,2,3-Trimethyl-4-(2-hydroxy-4-pyridyl-oxy)-6-nitro-chroman.

Beispiel 7

Ein Gemisch von 10 g 2,2-Dimethyl-4-(2-pyridyl-thio)-6-cyan-3-chromanol, 3 g Natriumhydroxid und 350 ml Dioxan wird 20 Min. gekocht. Man kühlt ab, filtriert, dampft das Filtrat ein und erhält 2,2-Dimethyl-4-(2-pyridylthio)-6-cyan-3-chromen, F. 110-112°.

Beispiel 8

Ein Gemisch von 2 g "B", 11,7 ml Ameisensäure und 3,3 ml Acetanhydrid wird 16 Std. bei 20° stehengelassen und anschließend 2 Std. auf 40-42° erwärmt. Nach Eindampfen und üblicher Aufarbeitung erhält man 2,2-Dimethyl-3-formyloxy-4-(6-hydroxy-3-pyridazinyl-oxy)-6-cyan-chroman.

Analog erhält man aus den entsprechenden 3-Hydroxychromanen:
2,2,3-Trimethyl-3-formyloxy-4-(6-hydroxy-3-pyridazinyl-oxy)-6-cyan-chroman
2,2-Dimethyl-3-formyloxy-4-(2-hydroxy-4-pyridyl-oxy)-6-cyan-chroman
2,2,3-Trimethyl-3-formyloxy-4-(2-hydroxy-4-pyridyl-oxy)-6-cyan-chroman.

Beispiel 9

Ein Gemisch von 1 g "B" und 5 ml Acetanhydrid wird 1 Std. gekocht. Man kühlt ab, arbeitet wie üblich auf und erhält 2,2-Dimethyl-3-acetoxy-4-(6-hydroxy-3-pyridazinyl-oxy)-6-cyan-chroman, F. 210-212°.

Analog erhält man:
2,2,3-Trimethyl-3-acetoxy-4-(6-hydroxy-3-pyridazinyl-oxy)-6-cyan-chroman
2,2-Dimethyl-3-acetoxy-4-(2-hydroxy-4-pyridyl-oxy)-6-cyan-chroman
2,2,3-Trimethyl-3-acetoxy-4-(2-hydroxy-4-pyridyl-oxy)-6-cyan-chroman.

Beispiel 10

Eine Lösung von 1 g 2,2-Dimethyl-4-(2-hydroxy-4-pyridyloxy)-6-nitro-3-chromanol in 25 ml Methanol wird bei 20° und 1 bar an 0,5 g 5%igem Pd-C bis zum Stillstand hydriert. Man filtriert, dampft ein und erhält 2,2-Dimethyl-4-(2-hydroxy-4-pyridyl-oxy)-6-amino-3-chromanol)

Analog erhält man:
2,2,3-Trimethyl-4-(2-hydroxy-4-pyridyl-oxy)-6-amino-3-chromanol
2,2-Dimethyl-4-(6-hydroxy-3-pyridazinyl-oxy)-6-amino-3-chromanol
2,2,3-Trimethyl-4-(6-hydroxy-3-pyridazinyl-oxy)-6-amino-3-chromanol.

Beispiel 11

Eine Lösung von 1 g 2,2-Dimethyl-4-(2-hydroxy-4-pyridyloxy)-6-amino-3-chromanol in 15 ml HCOOH und 1 ml Pyridin wird 19 Std. gekocht und eingedampft. Nach üblicher Aufarbeitung erhält man 2,2-Dimethyl-4-(2-hydroxy-4-pyridyloxy)-6-formamido-3-chromanol.

Beispiel 12

Ein Gemisch von 1 g 2,2-Dimethyl-4-(2-hydroxy-4-pyridyloxy)-6-amino-3-chromanol, 10 ml Acetanhydrid und 10 ml Pyridin wird 16 Std. bei 20° stehengelassen. Man dampft ein, reinigt chromatographisch und

erhält 2,2-Dimethyl4-(2-hydroxy-4-pyridyl-oxy)-6-acetamido-3-chromanol.

Beispiel 13

In eine siedende Lösung von 1 g "B" in 50 ml Methanol und 2 ml Wasser wird 14 Std. unter Rühren HCl eingeleitet. Man läßt erkalten und über Nacht stehen. Die ausgefallene 2,2-Dimethyl-4-(6-hydroxy-3-pyridazinyl-oxy)-3-chromanol-6-carbonsäure wird abfiltriert.

Beispiel 14

Ein Gemisch von 3,13 g "B", 31 g $Na_3PO_4.12\ H_2O$, 28 ml Pyridin, 28 ml Wasser, 67 ml Essigsäure und 25 g Raney-Ni (wasserfeucht) wird bei 20° 3 Std. gerührt. Nach Filtration arbeitet man wie üblich auf und erhält 2,2-Dimethyl-4-(6-hydroxy-3-pyridazinyl-oxy)-6-formyl-3-chromanol, F. 256-257°.

Analog erhält man:
2,2,3-Trimethyl-4-(6-hydroxy-3-pyridazinyl-oxy)-6-formyl-3-chromanol
2,2-Dimethyl-4-(2-hydroxy-4-pyridyl-oxy)-6-formyl-3-chromanol 2,2,3-Trimethyl-4-(2-hydroxy-4-pyridyl-oxy)-6-formyl-3-chromanol.

Beispiel 15

Man löst 3,13 g "B" in 40 ml tert.-Butanol und gibt unter Rühren 5,6 g gepulvertes KOH hinzu. Nach 1 Std. Kochen und üblicher Aufarbeitung erhält man 2,2-Dimethyl-4-(6-hydroxy-3-pyridazinyl-oxy)-6-carbamoyl-3-chromanol. Analog erhält man:
2,2,3-Trimethyl-4-(6-hydroxy-3-pyridazinyl-oxy)-6-carbamoyl-3-chromanol
2,2-Dimethyl-4-(2-hydroxy-4-pyridyl-oxy)-6-carbamoyl-3-chromanol
2,2,3-Trimethyl-4-(2-hydroxy-4-pyridyl-oxy)-6-carbamoyl-3-chromanol.

Beispiel 16

In eine Lösung von 3,12 g "A" in einem Gemisch von 20 ml Pyridin und 10 ml Triethylamin leitet man 5 Std. bei 20° $H_2S$ ein, dampft ein, arbeitet wie üblich auf und erhält 2,2-Dimethyl-4-(2-hydroxy-4-pyridyl-oxy)-6-thiocarbamoyl-3-chromanol, F. 242°.

Analog erhält man:
2,2,3-Trimethyl-4-(2-hydroxy-4-pyridyl-oxy)-6-thiocarbamoyl-chroman-3-ol.
2,2-Dimethyl-4-(6-hydroxy-3-pyridazinyl-oxy)-6-thiocarbamoyl-3-chromanol, F. 142-144°
2,2,3-Trimethyl-4-(6-hydroxy-3-pyridazinyl-oxy)-6-thiocarbamoyl-3-chromanol.

Beispiel 17

Ein Gemisch von 310 mg "B", 808 mg Lawesson-Reagenz und 50 ml Toluol wird 1 Std. unter $N_2$ gekocht. Übliche Aufarbeitung gibt 2,2-Dimethyl-4-(6-mercapto-3-pyridazinyloxy)-6-cyan-3-chromanol [2,2-Dimethyl-4-(1,6-dihydro-6-thioxo-3-pyridazinyl-oxy)-6-cyan-3-chromanol].

Analog erhält man aus "A" das 2,2-Dimethyl-4-(2-mercapto-4-pyridyl-oxy)-6-cyan-3-chromanol.

Analog erhält man:
2,2,3-Trimethyl-4-(6-mercapto-3-pyridazinyl-oxy)-6-cyan-3-chromanol
2,2-Dimethyl-4-(6-mercapto-3-pyridazinyl-oxy)-6-nitro-3-chromanol
2,2,3-Trimethyl-4-(6-mercapto-3-pyridazinyl-oxy)-6-nitro-3-chromanol
2,2-Dimethyl-4-(6-mercapto-3-pyridazinyl-oxy)-6-brom-3-chromanol
2,2,3-Trimethyl-4-(6-mercapto-3-pyridazinyl-oxy)-6-brom-3-chromanol
2,2,3-Trimethyl-4-(2-mercapto-4-pyridyloxy)-6-cyan-3-chromanol
2,2-Dimethyl-4-(2-mercapto-4-pyridyloxy)-6-nitro-3-chromanol 2,2,3-Trimethyl-4-(2-mercapto-4-pyridyloxy)-6-nitro-3-chromanol 2,2-Dimethyl-4-(2-mercapto-4-pyridyloxy)-6-nitro-3-chromanol
2,2,3-Trimethyl-4-(2-mercapto-4-pyridyloxy)-6-nitro3-chromanol.

Beispiel 18

Ein Gemisch von 312 mg "A", 20 ml Aceton, 400 mg $K_2CO_3$ und 0,2 ml Dimethylsulfat wird 2 Std. gekocht. Man filtriert, dampft ein und chromatographiert über Kieselgel. Mit Ethylacetat/Methanol (9:1) erhält man 2,2-Dimethyl4-(1,2-dihydro-1-methyl-2-oxo-4-pyridyl-oxy)-6-cyan-3-chromanol, F. 202-203°.

Analog erhält man:
2,2,3-Trimethyl-4-(1,2-dihydro-1-methyl-2-oxo-4-pyridyloxy)-6-cyan-3-chromanol
2,2-Dimethyl-4-(1,6-dihydro-1-methyl-6-oxo-3-pyridazinyloxy)-6-cyan-3-chromanol, F. 206-208°
2,2,3-Trimethyl-4-(1,6-dihydro-1-methyl-6-oxo-3-pyridazinyl-oxy)-6-cyan-3-chromanol, F. 197-199°
2,2-Dimethyl-4-(1,6-dihydro-1-methyl-6-oxo-3-pyridazinyloxy)-6-nitro-3-chromanol
2,2,3-Trimethyl-4-(1,6-dihydro-1-methyl-6-oxo-3-pyridazinyloxy)-6-nitro-3-chromanol
2,2-Dimethyl-4-(1,6-dihydro-1-methyl-6-oxo-3-pyridazinyloxy)-6-brom-3-chromanol
2,2,3-Triethyl-4-(1,6-dihydro-1-methyl-6-oxo-3-pyridazinyloxy)-6-brom-3-chromanol
2,2-Dimethyl-4-(1,2-dihydro-1-ethyl-2-oxo-4-pyridyloxy)-6-cyan-3-chromanol
2,2,3-Trimethyl-4-(1,2-dihydro-1-ethyl-2-oxo-4-pyridyloxy)-6-cyan-3-chromanol
2,2-Dimethyl-4-(1,2-dihydro-1-ethyl-6-oxo-3-pyridazinyloxy)-6-cyan-3-chromanol, F. 164-167°
2,2,3-Trimethyl-4-(1,2-dihydro-1-ethyl-6-oxo-3-pyridazinyloxy)-6-cyan-3-chromanol, F. 166-168°.

Beispiel 19

Ein Gemisch von 313 mg "B", 1 g $K_2CO_3$, 0,65 ml Dimethylsulfat und 16 ml DMF wird 3 Std. gekocht und wie üblich aufgearbeitet. Man erhält 2,2-Dimethyl-4-(6-methoxy-3-pyridazinyl-oxy)-6-cyan-3-chromanol, F. 224-227°.

Analog erhält man
2,2,3-Trimethyl-4-(6-methoxy-3-pyridazinyl-oxy)-6-cyan-3-chromanol
2,2-Dimethyl-4-(2-methoxy-4-pyridyl-oxy)-6-cyan-3-chromanol
2,2,3-Trimethyl-4-(2-methoxy-4-pyridyl-oxy)-6-cyan-3-chromanol.

Beispiel 20

Analog Beispiel 18 erhält man aus "B" und 2-Brompropan das 2,2-Dimethyl-4-(1,6-dihydro-1-isopropyl-6-oxo-3-pyridazinyloxy)-6-cyan-3-chromanol, F. 201-203°.

Beispiel 21

a) Ein Gemisch von 5 g "B", 5 g (+)-Campher-10-sulfonsäurechlorid und 50 ml Pyridin wird 5 Std. auf 70° erwärmt. Man arbeitet mit verdünnter Salzsäure und Ethylacetat wir üblich auf, trennt chromatographisch an Kieselgel mit Dichlormethan/Ethylacetat-Gemischen und erhält zwei Epimere des "B"-(+)-campher-10sulfonsäureesters, F. 223-224° und F. 127-150°.

b) Ein Gemisch von 2 g des "unpolaren" Epimeren (F. 223-224°), 16 g "Natriumhydroxid auf Träger" (E. Merck, Katalog "Reagenzien, Diagnostica, Chemikalien", 1987/88, Seite 587, Nr. 1567) und 80 ml Methanol wird 20 Std. bei 20° gerührt. Man dampft ein, löst in Wasser, gibt HCl bis pH 8 hinzu und filtriert das erhaltene 2,2-Dimethyl-4-(1,6-dihydro-6-oxo-3-pyridazinyloxy)-6-cyan-3-chromen (F. 226-228°) ab. Aufarbeitung des Filtrats mit Salzsäure/Ethylacetat bei pH 4 und Chromatographie an Kieselgel mit Dichlormethan/Ethylacetat/Methanol liefert (-)-2,2-Dimethyl-4-(1,6-dihydro-6-oxopyridazinyl-oxy)-6-cyan-3-chromanol, F. 229°; [α] -168,5°.

c) Analog erhält man aus dem "polaren" Epimeren (F. 127-150°) das (+)-2,2-Dimethyl-4-(1,6-dihydro-6-oxo-3-pyridazinyl-oxy)-6-cyan-3-chromanol, F. 232-233°; [α] +170,0°.

Analog erhält man aus "A" über die entsprechenden (+)Camphersulfonsäureester das 2,2-Dimethyl-4-(2-hydroxy-4-pyridyl-oxy)-6-cyan-3-chromen (F. 263-264°) sowie (-)und (+)-2,2-Dimethyl-4-(2-hydroxy-4-pyridyl-oxy)-6-cyan-3-chromanol.

Analog erhält man aus 2,2-Dimethyl-4-(1,6-dihydro-1-methyl-6-oxo-3-pyridazinyl-oxy)-6-cyan-3-chromanol über die entsprechenden (+)-Camphersulfonsäureester das 2,2-Dimethyl-4-(1,6-dihydro-1-methyl-6-oxo-3-pyridazinyl-oxy)-6-cyan-3-chromen (F. 144-146°) sowie (-)-2,2-Dimethyl-4R-(1,6-dihydro-1-methyl-6-oxo-3-pyridazinyl-oxy)-6-cyan-3S-chromanol (F. 164°; [α] -165,1°) und (+)-2,2-Dimethyl-4S-(1,6-dihydro-1-methyl-6-oxo-3-pyridazinyl-oxy)-6-cyan-3R-chromanol (F. 161-162°; [α] +171,8°).

Analog erhält man aus 2,2,3-Trimethyl-4-(1,6-dihydro-6-oxo-3-pyridazinyl-oxy)-6-cyan-3-chromanol über die entsprechenden (+)-Camphersulfonsäureester das 2,2,3-Trimethyl-4-(1,6-dihydro-6-oxo-3-pyridazinyl-oxy)-6-cyan-3-chromen sowie (-)-2,2,3-Trimethyl-4-(1,6-dihydro 6-oxo-3-pyridazinyl-oxy)-6-cyan-3-chromanol (F. 131-134°; [α] -222,2°) und (+)-2,2,3-Trimethyl-4-(1,6-dihydro-6-oxo-3-pyridazinyl-oxy)-6-cyan-3-chromanol (F. 131-134°; [α] +222,2°).

Die nachstehenden Beispiele betreffen pharmazeutische Zubereitungen, die Verbindungen der Formel I oder ihre physiologisch unbedenklichen Salze enthalten:

Beispiel A Tabletten

Ein Gemisch von 1 g 2,2-Dimethyl-4-(6-hydroxy-3-pyridazinyl-oxy)-6-cyan-3-chromanol 4 kg Lactose 1,2 kg Kartoffelstärke, 0,2 kg Talk und 0,1 kg Magnesiumstearat wird in üblicher Weise zu Tabletten gepreßt, derart, daß jede Tablette 0,1 mg Wirkstoff enthält.

Beispiel B Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C Kapseln

Man füllt 1 kg 2,2,3-Trimethyl-4-(6-hydroxy-3-pyridazinyloxy)-6-cyan-3-chromanol in üblicher Weise in Hartgelatinekapseln, so daß jede Kapsel 0,5 mg Wirkstoff enthält.

Beispiel D Ampullen

Eine Lösung von 10 g 2,2-Dimethyl-4-(6-hydroxy-3-pyridazinyl-oxy)-6-cyan-3-chromanol in 70 1 1,2-Propandiol wird mit zweifach destilliertem Wasser auf 100 1 aufgefüllt, steril filtriert, in Ampullen abgefüllt und steril verschlossen. Jede Ampulle enthält 0,1 mg Wirkstoff.

Analog sind Tabletten, Dragees, Kapseln oder Ampullen erhältlich, die einen oder mehrere der übrigen Wirkstoffe der Formel I und/oder ihrer physiologisch unbedenklichen Salze enthalten.

**Ansprüche**

1. Chromanderivate der Formel I

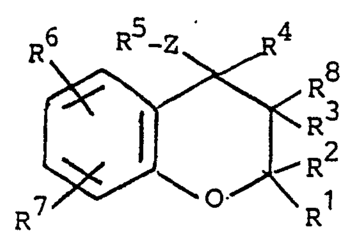

I

worin

$R^1$ A,

$R^2$ und $R^8$ jeweils H oder A,

$R^1$ und $R^2$ zusammen auch Alkylen mit 3-6 C-Atomen,

$R^3$ H, OH, OA oder OAc,

$R^4$ H,

$R^3$ und $R^4$ zusammen auch eine Bindung,

$R^5$ einen unsubstituierten oder ein- oder zweifach durch A, F, Cl, Br, J, OH, OA, OAc, SH, $NO_2$, $NH_2$, AcNH, HOOC und/oder AOOC substituierten Pyridyl-, Pyridazinyl-, Pyrimi dinyl- Pyrazinyl-, Oxo-dihydro-pyridyl-, Oxo-dihydro-pyridazinyl-, Oxo-dihydropyrimidinyl- oder Oxo-dihydro-pyrazinylrest,

$R^6$ und $R^7$ jeweils H, A, HO, AO, CHO, ACO, ACS, HOOC, AOOC, AO-CS, ACOO, A-CS-O, Hydroxyalkyl mit 1-6 C-Atomen, Mercaptoalkyl mit 1-6 C-Atomen, $NO_2$, $NH_2$, NHA, $NA_2$, CN, F, Cl, Br, J, $CF_3$, ASO, $ASO_2$, AO-SO, AO-$SO_2$, AcNH, AO-CO-NH, $H_2NSO$, HANSO, $A_2NSO$, $H_2NSO_2$, $HANSO_2$, $A_2NSO_2$, $H_2NCO$, HANCO, $A_2NCO$, $H_2NCS$, HANCS, $A_2NCS$, ASONH, $ASO_2NH$, AOSONH, $AOSO_2NH$, ACO-alkyl, Nitroalkyl, Cyanalkyl, A-C(=NOH) oder A-C(=$NNH_2$),

Z O, S, oder NH,

A Alkyl mit 1-6 C-Atomen,

alkyl Alkylen mit 1-6 C-Atomen und

Ac Alkanoyl mit 1-8 C-Atomen oder Aroyl mit 7-11 C-Atomen

bedeuten,

sowie deren Salze.

2.

a) 2,2-Dimethyl-4-(2-hydroxy-4-pyridyl-oxy)-6-cyan-3-chromanol;

b) 2,2,3-Trimethyl-4-(2-hydroxy-4-pyridyl-oxy)-6-cyan-3-chromanol;

c) 2,2-Dimethyl-4-(6-hydroxy-3-pyridazinyl-oxy)-6-cyan-3-chromanol;

d) 2,2,3-Trimethyl-4-(6-hydroxy-3-pyridazinyl-oxy)-6-cyan-3-chromanol;

e) 2,2-Dimethyl-4-(1,6-dihydro-1-methyl-6-oxo-3-pyridazinyl-oxy)-6-cyan-3-chromanol;

f) 2,2,3-Trimethyl-4-(1,6-dihydro-1-methyl-6-oxo-3-pyridazinyl-oxy)-6-cyan-3-chromanol.

3. Verfahren zur Herstellung von Chromanderivaten der Formel I gemäß Anspruch 1, dadurch gekennzeichnet, daß man ein Chroman der Formel II

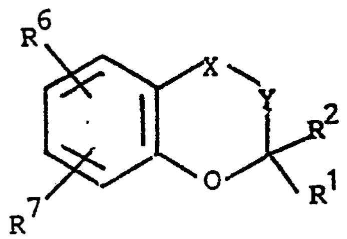

II

worin

X-Y

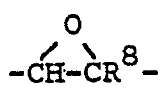

oder -CHE-$CR^3$-$R^8$- und

E Cl, Br, J oder eine reaktionsfähig veresterte OH-Gruppe bedeuten und

$R^1$, $R^2$, $R^3$, $R^6$, $R^7$ und $R^8$ die bei Formel I angegebenen Bedeutungen haben,

mit einer Verbindung der Formel III

$R^5$-ZH III

worin $R^5$ und Z die bei Formel I angegebenen Bedeutungen haben,

oder mit einem ihrer reaktionsfähigen Derivate umsetzt

und/oder daß man eine Verbindung der Formel I, worin $R^3$ OH und $R^4$ H bedeutet, dehydratisiert und/oder daß man in einer Verbindung der Formel I einen oder mehrere der Reste $R^3$, $R^5$, $R^6$ und/oder. $R^7$ in andere Reste $R^3$, $R^5$, $R^6$ und/oder $R^7$ umwandelt und/oder daß man eine basische Verbindung der Formel I durch Behandeln mit einer Säure in eines ihrer Säureadditionssalze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I zur Bekämpfung von Krankheiten.
7. Verwendung von Verbindungen der Formel I zur Herstellung eines Arzneimittels.
8. Verwendung von Verbindungen der Formel I bei der Bekämpfung von Krankheiten.

Europäisches Patentamt

# EUROPÄISCHER RECHERCHENBERICHT

Nummer der Anmeldung

EP 89 11 8755

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 273 262 (MERCK)<br>* Seite 16, Anspruch 1; Seite 17, Ansprüche 5,6; Seite 2, Zeilen 44-54 *<br>--- | 1,5 | C 07 D 405/12<br>A 61 K 31/44 //<br>(C 07 D 405/12<br>C 07 D 311:00<br>C 07 D 213:00 )<br>(C 07 D 405/12<br>C 07 D 311:00<br>C 07 D 237:00 )<br>(C 07 D 405/12<br>C 07 D 311:00<br>C 07 D 239:00 )<br>(C 07 D 405/12<br>C 07 D 311:00<br>C 07 D 241:00 ) |
| Y | EP-A-0 176 689 (BEECHAM)<br>* Seite 1, Anspruch 1 *<br>--- | 1,5 | |
| P,A | GB-A-2 204 868 (SANDOZ)<br>* Seite 32, Anspruch 1; Seiten 42,43, Ansprüche 9,10,12 *<br>--- | 1,5 | |
| A | JOURNAL OF MEDICINAL CHEMISTRY<br>Band 29, Nr. 11, 1986, Seiten 2194-2201; J.M. EVANS et al.: "Synthesis and Antihypertensive Activity of 4-(Cyclic amido)-2H-1-benzopyrans" * Seite 2195, Tabelle I, Verbindungen 52-56; Seite 2197, Tabellen III,IV *<br>--- | 1,5 | |
| Y | CHEMICAL ABSTRACTS<br>Band 99, Nr. 5, 1. August 1983, Seite 18, Spalte 1, Zusammenfasssung Nr. 32772p, Columbus, Ohio, USA; K. KOU et al.: "The effects of 3,4-dihydro-8-(2-hydroxy-3-isopropylaminopropoxy)-3-nitroxy-2H-1-benzopyran (K-351) and its denitrated derivatives on smooth muscle cells of the dog coronary artery" & Br. J. Pharmacol. 1983, Band 79, Nr. 1, Seiten 285-295<br>--- -/- | 1,5 | RECHERCHIERTE SACHGEBIETE (Int. Cl.5)<br>C 07 D 311/00<br>C 07 D 405/00 |

Der vorliegende Recherchenbericht wurde für alle Patentansprüche erstellt

| Recherchenort | Abschlußdatum der Recherche | Prüfer |
|---|---|---|
| BERLIN | 12-12-1989 | KYRIAKAKOU G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 89 11 8755

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | CHEMICAL ABSTRACTS Band 84, Nr. 5, 2. Februar 1976, Seite 451, Spalte 1, Zusammenfassung Nr. 30936m, Columbus, Ohio, USA; S.M. EL-ANTABLY et al.: "Synthesis and blood pressure lowering activity of benzylic ethers of 2-diethylaminoethanol and a related diamine" & J. Pharm Sci. 1975, Band 64, Nr. 8, Seiten 1423-1425 --- | 1,5 | |
| A | JOURNAL OF THE CHEMICAL SOCIETY Perkin Transactions I, Nr. 12, 1983, Seiten 2903-2912, London, GB; B.R. BROWN et al.: "Synthesis and Reactions of 4-Aryloxyflavans" * Seite 2905, Verbindungen 9,11,13 * ----- | 1 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| BERLIN | 12-12-1989 | KYRIAKAKOU G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)